# EUROPÄISCHE PATENTANMELDUNG

(11) **EP 4 303 307 A1**
(43) Veröffentlichungstag der Anmeldung: **10.01.2024**
(21) Anmeldenummer: 23000055.6
(22) Anmeldetag: 06.04.2023
(51) Int. Cl.: C12P 7/22, C12P 7/24, C12P 11/00, C12N 9/10, C12R 1/645

(54) **VERFAHREN ZUR HERSTELLUNG VON AROMASTOFFEN UNTER VERWENDUNG EINER O-METHYLTRANSFERASE AUS PLEUROTUS**

(30) Priorität: 07.04.2022 EP 22167232
(71) Anmelder: Justus-Liebig-Universität Gießen Rechtsabteilung, 35390 Gießen (DE)
(72) Erfinder: Zorn, Holger, 35435 Wettenberg (DE); Rühl, Martin, 61209 Echzell (DE); Brescia, Fabio Francesco, 65510 Idstein (DE)
(74) Vertreter: Stumpf, Peter

(57) **Zusammenfassung**

Die Erfindung betrifft ein Verfahren zum Herstellen mindestens eines Aromastoffes mit O-Methyltransferase aus *Pleurotus sapidus* umfassend die Schritte

• a) Bereitstellung eines Umsetzungsmediums,
• b) Einbringen von mindestens einem Edukt und O-Methyltransferase in das Umsetzungsmedium aus Schritt a),
• c) Umsetzung des mindestens einen Eduktes unter Katalyse von O-Methyltransferase zu dem mindestens einen Aromastoff, wobei Hydroxy- und Thiolgruppen zu Methoxy- und Thioethergruppen umgesetzt werden, gezeigt an Hydroxybenzaldehyd, Hydroxybenzylalkohol, 2-Methyl-3-furanthiol, Vanillin und 6-Hydroxycoumarin, wobei Anisaldehyd, Veratrumaldehyd und 6-Methoxycoumarin entstehen.

## Beschreibung

Die vorliegende Erfindung betrifft ein Verfahren zur Gewinnung eines von einem Basidiomyceten, insbesondere von *Pleurotus* spp. wie z. B. *Pleurotus sapidus, Pleurotus eryngii, Pleurotus pulmonarius, Pleurotus sayor-caju, Pleurotus floridanus, Pleurotus albanus, Pleurotus djamor, Pleurotus ostreatus, Pleurotus calyptratus, Pleurotus citrinopileatus, Pleurotus flabellatus, Pleurotus salmoneo-stramineus, Pleurotus comucopiae, Pleurotus dryinus*, *Pleurotus cystidiosus, Pleurotus spodoleucus, Pleurotus populinus, Pleurotus euosmus, Pleurotus nebrodensis, Pleurotus geesterani, Pleurotus geesterani* oder *Pleurotus ostreatus var. columbinus,* produzierten Enzyms O-Methyltransferase in einem Wirtsorganismus, wie z. B. *Escherichia coli* (*E. coli*). Insbesondere betrifft die vorliegende Erfindung die Verwendung des Enzyms O-Methyltransferase zur Umsetzung von Aroma-Präkursoren mit Hydroxy- oder Thiolgruppen zu den entsprechenden Methoxy- und Thioether-Verbindungen.

### Stand der Technik

*Aromastoffe* sind chemische Verbindungen, die über die Geruchs- und/oder Geschmacksrezeptoren Geruchs- und/oder Geschmackseindrücke bei Menschen und/oder Säugetieren hervorrufen. *Aromastoffe* können - wie beispielsweise Vanillin - zu der chemischen Substanzgruppe der sogenannten *aromatischen Verbindungen* gehören, also chemischen Substanzen, die als Strukturelement einen oder mehrere Benzolringe enthalten. Dem Fachmann ist bekannt, dass der Begriff *Aromastoff* alle Stoffe bzw. Substanzen bezeichnet, die Geruchs- und/oder Geschmackseindrücke hervorrufen, ohne dass damit ein konkreter Bezug zur chemischen Struktur des Aromastoffs besteht. Dem Fachmann ist weiterhin bekannt, dass unabhängig davon die Begriffe *Aromat, aromatische Verbindung, aromatisches Substrat* usw. sich auf die Struktur einer Substanz/chemischen Verbindung beziehen und kennzeichnen, dass die betreffende Substanz als Strukturelement einen oder mehrere Benzolringe aufweist. Aufgrund dieser allgemein bekannten Bedeutungen kann der Fachmann aus dem Textzusammenhang eindeutig zwischen *Aromastoff* und *aromatischer Verbindung* usw. unterscheiden, sowie auch erkennen, wann etwa eine Substanz/Verbindung beide Eigenschaften aufweist.

Die Verwendung von Aromastoffen erstreckt sich auf zahlreiche Bereiche des täglichen Lebens. So werden die Verbindungen neben deren Verwendung in Lebensmitteln wie Süßwaren, Erfrischungsgetränken, feinen Backwaren oder Fertiggerichten beispielsweise in kosmetischen Produkten, Arzneimitteln sowie Wasch- und Reinigungsmitteln eingesetzt. Als Beispiel sei hier die Verbindung 4-Methoxybenzaldehyd, auch unter dem Trivialnamen p-Anisaldehyd oder Anisaldehyd bekannt, aufgeführt. Der Aromastoff besitzt einen süßlichen, blumigen und Anis-ähnlichen Geruch. Natürliche Vorkommen erstrecken sich unter anderem auf Anis, Basilikum, Fenchel, Sternanis, Vanille, sowie Zimt. Die Verbindung wird unter anderem in alkoholischen und nicht-alkoholischen Getränken, Backwaren, Milchprodukten sowie verschiedenen Süßwaren eingesetzt.

Aufgrund zunehmender Vorbehalte von Verbrauchern gegenüber synthetischen Lebensmittelinhaltsstoffen ist der Bedarf an insbesondere natürlichen Aromastoffen steigend. Um dieser Nachfrage gerecht zu werden, rücken neben klassisch extraktiven Verfahren aus Pflanzen zunehmend biotechnologische Verfahren in den Fokus der Aromaforschung. Gemäß der Verordnung (EG) Nr. 1334/2008 werden natürliche Aromastoffe dabei als "Aromastoff, durch geeignete physikalische, enzymatische oder mikrobiologische Verfahren aus pflanzlichen, tierischen oder mikrobiologischen Ausgangsstoffen gewonnen..." definiert. Der Einsatz von Pilzen der Abteilung *Basidiomycota* sowie deren Enzymsystem zur industriellen Produktion natürlicher Aromastoffe stellt damit eine umweltfreundliche und perspektivreiche Alternative zur chemischen Synthese und extraktiven Verfahren dar. Hierdurch kann auch der Einsatz von teilweise toxischen Substanzen und organischen Lösungsmitteln, die im Zuge der chemischen Synthese benötigt werden, minimiert werden. Die chemische Synthese von *p*-Anisaldehyd, eine Substanz, die sowohl eine aromatische Verbindung ist, als auch ein erwünschter Aromastoff, erfolgt bspw. durch Methylierung und Oxidation von *p*-Cresol oder durch Oxidation von Anethol auf klassisch-chemischem Wege. Damit sind mehrere nachteilige Aspekte verbunden, z. B. der Einsatz teils toxischer Substanzen (z. B. *p*-Cresol) sowie teurer Katalysatoren. Auch der Energieaufwand für die Durchführung üblicher chemischer Synthesen ist sehr hoch und es entstehen Nebenprodukte sowie Abfälle (z. B. organische Lösungsmittel), die kostspielig regeneriert oder entsorgt werden müssen.

Neben den natürlichen Vorkommen und der chemischen Synthese ist die Bildung von p-Anisaldehyd für einige Pilze der Abteilung *Basidiomycota* bekannt. Hierzu zählen neben *Bjerkandera adusta* auch Vertreter der Gattung *Pleurotus* wie beispielsweise *P. ostreatus, P. pulmonarius* sowie *P*. *sapidus.* Die Biogenese der Verbindung wird dabei mit dem Lignin-Abbau in Verbindung gebracht. Es wird postuliert, dass im Zuge der Biogenese von *p*-Anisaldehyd der Präkursor *p*-Hydroxybenzaldehyd auftritt. Für die enzymatische Katalyse der Übertragung einer Methylgruppe auf eine Hydroxygruppe zur Generierung des entsprechenden Methoxy-Derviates sind in der Literatur die zur Klasse der Transferasen gehörenden *O*-Methyltransferasen beschrieben.
Analog zu der Methylierung von Substraten mit Hydroxygruppen im Zuge der Bildung von Aromastoffen wie *p*-Anisaldehyd erfolgt auch bei der Biogenese bestimmter schwefelhaltiger Aromastoffe die Übertragung einer Methylgruppe. Als Beispiel sei hier die Methylierung von 2-Methyl-3-furanthiol zu 2-Methyl-3-(methylthio)furan genannt. Schwefelhaltige Aromastoffe werden oftmals mit fleischartigen Geruchsattributen assoziiert. So weist die Verbindung 2-Methyl-3-furanthiol ein Aroma auf, das als fleischig beschrieben wird. Das entsprechende ,Thioether-Derivat besitzt ähnliche Geruchseigenschaften, bietet allerdings den Vorteil, dass keine Disulfidbrücken ausgebildet werden können. Die enzymatische Bildung von 2-Methyl-3-(methylthio)furan ausgehend von 2-Methyl-3-furanthiol wurde durch eine in der Rattenleber vorkommende S-Methyltransferase beschrieben. Für Ständerpilze wurde bislang kein entsprechendes Enzym beschrieben. Für die chemische Synthese von 2-Methyl-3-(methylthio)furan ausgehend von 2-Methyl-3-furanthiol wurde beispielsweise die Umsetzung mit der giftigen und karzinogen Verbindung Dimethylsulfat offenbart.

### Aufgabe

Aufgabe der vorliegenden Erfindung ist es, die vorstehend beschriebenen Nachteile des Standes der Technik bei der Herstellung Methoxy-substituierter und/oder Thiomethyl-substituierter Aromastoffe zu beheben.

### Lösung der Aufgabe

Gelöst wird diese Aufgabe durch die Verwendung des Enzyms O-Methyltransferase zur Umsetzung von Aroma-Präkursoren mit Hydroxy- oder Thiolgruppen zu den entsprechenden Methoxy- und Thioether-Verbindungen gemäß den Ansprüchen.

Das Enzym *O*-Methyltransferase wird dabei aus einem Basidiomyceten, wie z. B. *Pleurotus sapidus*, *Pleurotus eryngii, Pleurotus pulmonarius*, *Pleurotus sayor-caju, Pleurotus florida, Pleurotus albanus, Pleurotus djamor, Pleurotus ostreatus, Pleurotus calyptratus, Pleurotus citrinopileatus, Pleurotus flabellatus*, *Pleurotus salmoneo-stramineus, Pleurotus comucopiae, Pleurotus dryinus, Pleurotus cystidiosus*, *Pleurotus spodoleucus, Pleurotus populinus, Pleurotus euosmus, Pleurotus nebrodensis, Pleurotus geesterani, Pleurotus geesterani* oder *Pleurotus ostreatus var. Columbinus* isoliert oder rekombinant in einem Wirtsorganismus, wie z. B. *Escherichia coli (E. coli),* hergestellt, oder durch Cokultivierung erzeugt.

Ausgangspunkt war es, in Ständerpilzen der Abteilung *Basidiomycota,* wie bspw. *Pleurotus sapidus,* vorhandene *O*-Methyltransferasen zu identifizieren und ein Verfahren für die Produktion des Enzyms in einem Wirtsorganismus zu etablieren. So konnte das Enzym für die Methylierung von Aromapräkursoren mit Hydroxy- bzw. Thiol-Gruppen eingesetzt werden.

### Vorteil der Erfindung

Die vorliegende Erfindung ermöglicht die biokatalytische Umsetzung der Methylierungsreaktion in einem geeigneten Medium, die zu Methoxy-substituierten und/oder Thiomethyl-substituierten Aromastoffen führt. Es wird somit ein enzymkatalysiertes Herstellungsverfahren für Aromastoffe bereitgestellt, das von aromatischen Verbindungen mit Hydroxyl- und/oder Thiolgruppen ausgeht und zu entsprechenden methylierten Verbindungen führt. Ein geeignetes Umsetzungsmediu m ist eine wässrige Lösung, Suspension, Dispersion, Zellaufschlämmung usw. in der die Umsetzung, d. h. die vom Biokatalysator katalysierte chemische Reaktion oder Reaktionsfolge, stattfindet. Der Einsatz entsprechender Enzyme als Biokatalysator erfüllt die Prinzipien der *Green Chemistry*, da hierdurch unter anderem Abfall minimiert, die Atomökonomie beachtet, ungefährlichere Syntheserouten angewendet, sicherere Chemikalien und Lösungsmittel verwendet, sowie auch der Energieverbrauch im Vergleich zu den im Stand der Technik genannten Synthesen minimiert werden kann. Mit dem Begriff Biokatalysator werden hier Enzyme verstanden, insbesondere O-Methyltransferasen. Der Fachmann kennt Methyltransferasen oder Methylasen als Enzyme der Kategorie EC 2.1.1., die Substrate methylieren, d. h. die Übertragung von Methylgruppen auf ein geeignetes Akzeptorsubstrat katalysieren.
Entsprechende Enzyme kommen universell in Lebewesen vor und sind an der Regulation unterschiedlichster Stoffwechselvorgänge beteiligt. Unterschieden wird hierbei prinzipiell zwischen *O*-Methyltransferasen, *N*-Methyltransferasen, *C*-Methyltransferasen sowie die seltener vorkommenden *S*- und Halogenid-Methyltransferasen.

Der Umfang der Erfindung ist nicht beschränkt auf die Verwendung einer O-Methyltransferase aus *Pleurotus sapidus, Pleurotus eryngii, Pleurotus pulmonarius*, *Pleurotus sayor-caju, Pleurotus floridanus, Pleurotus albanus, Pleurotus djamor, Pleurotus ostreatus, Pleurotus calyptratus, Pleurotus citrinopileatus, Pleurotus flabellatus, Pleurotus salmoneo-stramineus*, *Pleurotus comucopiae, Pleurotus dryinus*, *Pleurotus cystidiosus, Pleurotus spodoleucus, Pleurotus populinus, Pleurotus euosmus, Pleurotus nebrodensis, Pleurotus geesterani, Pleurotus geesterani* oder *Pleurotus ostreatus var. columbinus.* Die Verwendung von *O*-Methyltransferasen aus weiteren *Pleurotus*-Arten ist ebenfalls vom Umfang der Erfindung umfasst, denn selbst dann, wenn die Sequenzabweichung gegenüber anderen bereits bekannten Sequenzen nur wenige Prozent beträgt (z. B. lediglich 1-5%), treten bekannterma-βen - insbesondere, wenn sie im Bereich des aktiven Zentrums eines Enzyms auf treten - unvorhersehbare, überraschende und weitreichende Eigenschaftsänderungen auf.

Die Enzymklasse der *O*-Methyltransferasen ist in der Natur zwar weit verbreitet, jedoch ist sowohl das Vorkommen in *Pleurotus spp.* als auch die Verwendung von *O-*Methyltransferasen aus *Pleurotus spp.* für Synthesezwecke bisher gänzlich unbekannt. Beispielsweise wird bei S. A. Heleno et al., "Bioactivity of phenolic acids: Metabolitesv ersus parent compounds: A review", in Food Chemistry, Bd.173 (2015), 501-513 zwar in dortiger Tabelle 1 auf die Relevanz diverser *O*-Methyltransferasen für die natürlichen Biosynthesewege diverser phenolischer Säuren hingewiesen, aber es wird bezüglich keines einzigen der aufgeführten Enzyme auch nur angedeutet, dass es in einer *Pleurotus spp.* vorkommt. Tabelle 2 in S. A. Heleno et al. offenbart lediglich, dass Aromastoffe wie beispielsweise Vanillinsäure in *Pleurotus* spp. vorkommen. Es wird dort nicht offenbart, dass auch in Biosynthesewegen sekundärer Inhaltsstoffe von *Pleutotus spp. O*-Methyltransferasen involviert sind.

Bei den von Heleno et al. untersuchten Produkten handelt es sich zudem um methoxylierte Carbonsäuren, die bekanntermaßen nicht als Aromastoffe gelten (z. B. Vanillesäure, Veratrumsäure, Syringasäure, Sinapinsäure). Da derartige Verbindungen freie Carbonsäuregruppen aufweisen, handelt es sich um nichtflüchtige Verbindungen, die nicht in der Lage sind, gasförmig Geruchsrezeptoren zu erreichen, und daher per se nicht in der Lage sind, zu Aromen beizutragen.

Die Verwendung von *O*-Methyltransferasen für präparative technische Synthesen wird bei S. A. Heleno an keiner Stelle offenbart, oder auch nur nahegelegt. S. A. Heleno et al. offenbaren in Abschnitt 6 bezüglich Methylierung von sekundären Inhaltsstoffen wie beispielsweise Vanillinsäure ausschließlich die klassische chemische Synthese; enzymbasierte Synthesen werden dort nur als Teilschritte in natürlichen Biosynthesewegen beschrieben.

*O*-Methyltransferasen werden für wissenschaftliche und technische Zwecke aus unterschiedlichsten Quellen gewonnen. US 2015/0322465 A1 offenbart beispielsweise die Verwendung einer aus Pflanzen gewonnenen *O*-Methyltransferase für die Herstellung von z. B. Vanillin. EP 2 749 644 B1 offenbart eine *O*-Methyltransferase aus *Medicago sativa* oder *Vanilla planifolia* für einen Multienzymkomplex, ebenfalls zur Herstellung von Vanillin.

Es sind jedoch bisher aus dem Stand der Technik keine *O*-Methyltransferasen aus *Pleurotus* spp. bekannt. Zwar ist aus der Genomsequenzierung von *P. ostreatus* eine Proteinsequenz bekannt (vgl. Online-Datenbank UniProt, Eintrag vom 3. September 2014, XP55964860, Datenbank-Zugriff Nr. AOA067NAT9), bei der es sich - soweit bisher erkennbar - möglicherweise um die Proteinsequenz für ein Mitglied einer Klasse-I-artigen SAM-bindenden Methyltransferasen Überfamilie handeln könnte (bzw. ein Mitglied einer Kationen-unabhängigen *O*-Methyltransferasen-Familie). Da die Proteinsequenz lediglich aus der Genomsequenzierung von *P*. *ostreatus* automatisiert erstellt und auf UniProt (Universal Protein Knowledgebase) eingestellt wurde, kann allein dadurch nicht davon ausgegangen werden, dass die entsprechende *O*-Methyltransferase auch wirklich funktionell exprimiert wird bzw. aktiv ist. Diese Sequenz wurde lediglich automatisiert erstellt und in der Datenbank abgelegt, ohne jeglichen Beleg dafür, dass das entsprechende Enzym wirklich exprimiert wird und existent ist. Dieser automatisiert erstellte Datenbank-Eintrag weist zudem explizit den Hinweis auf, dass die publizierte Proteinsequenz konservierte Regionen nicht enthält, die bekanntermaßen für die sichere Zuordnung der Funktionalität zwingend erforderlich sind. Weiterhin ist der Eintrag bei UniProt als *unreviewed* (nicht überprüft) eingestuft. Es muss daher davon ausgegangen werden, dass dieser Datensatz keinen validen Stand der Technik darstellt.

Aus dem Stand der Technik sind somit bisher keine *O*-Methyltransferasen aus *Pleurotus spp.* bekannt.

Der Umfang der Erfindung ist weiterhin nicht beschränkt durch die hier beispielhaft offenbarten Ausführungsbeispiele. In der nachfolgenden Beschreibung umfassen beispielsweise Bereichsangaben stets alle - auch nicht explizit genannte - innerhalb der genannten Intervallgrenzen liegenden Zwischenwerte und alle denkbaren Teilintervalle.

### Ausführungsbeispiele

### 1. Beispielhafte Kultivierung von Pleurotus sapidus-Stämmen zur Isolierung von Aromastoffen und Identifizierung von O-Methyltransferasen

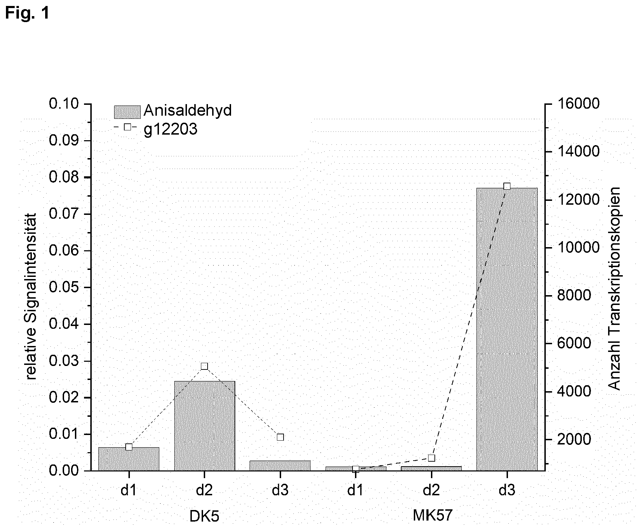

Beispielhaft werden zwei verschiedene Stämme von *Pleurotus sapidus* verwendet (ein monokaryotischer Stamm (MK57) und ein dikaryotischer (DK) Stamm). Dem Fachmann sind die Rahmenbedingungen zur Kultivierung von *Pleurotus sapidus-*Stämmen bekannt. Beispielhaft erfolgt dies in Submerskultur unter Verwendung von Malzextrakt-Sojapepton-Medium (30 g Malzextrakt, 3 g Sojapepton; pro Liter Reinstwasser; pH 5,6). Dazu werden Triplikate angesetzt und drei Tage kultiviert. An jedem Kulturtag wird für die Analyse des entsprechenden Triplikates das Myzel vom Kulturüberstand separiert (3000 g, 10 min, 4 °C). Das Myzel wird bei -80 °C gelagert. Von dem Kulturüberstand werden 5 mL in geeignete Gefäße z. B. sterile 20-mL-Headspacevials transferiert und bei -20 °C gelagert. Unmittelbar vor der Extraktion der Aromastoffe werden die Kulturüberstände aufgetaut und 50 µL interner Standard (Thymol-Lösung (3,144 * 10⁻³ mg mL⁻¹)) jeweils zu den Proben gegeben. Die Extraktion der Aromastoffe erfolgt nach gängigen Methoden, im vorliegenden Ausführungsbeispiel beispielhaft mittels *stir bar sorptive extraction* (1 h, 1000 rpm, 4 °C) und die Analyse mittels GC-MS. Andere Extraktionsmethoden sind ebenfalls anwendbar, insbesondere präparative Methoden für die Gewinnung größerer Substanzmengen an gewünschtem Aromastoff. Nach erfolgter Auswertung der gaschromatographischen Daten wird das Myzel von *Pleurotus sapidus* für die RNA-Extraktion mittels TRIzol verwendet. Die RNA-Sequenzierung erfolgt durch beliebige im Stand der Technik bekannte Verfahren.
Im Zuge der Auswertung der gaschromatographischen Daten wird gezeigt, dass der dikaryotische Stamm und insbesondere der monokaryotischer Stamm den Aromastoff p-Anisaldehyd produzieren (vgl. Fig. 1). Die Daten der Aromaanalytik korrelieren mit einem für eine *O*-Methyltransferase codierenden Gen. Dies belegt eine Beteiligung des Enzyms *O*-Methyltransferase in der Biogenese von p-Anisaldehyd.
Das vorstehend beispielhaft anhand zweier Stämme von *Pleurotus sapidus* beschriebene Verfahren zur Kultivierung ist analog auch für die Kultivierung von Stämmen anderer Subspezies der Gattung Pleurotus, wie z.B. *Pleurotus eryngii, Pleurotus pulmonarius, Pleurotus sayor-caju, Pleurotus floridanus*, *Pleurotus albanus* und *Pleurotus djamor, Pleurotus ostreatus, Pleurotus calyptratus, Pleurotus citrinopileatus, Pleurotus flabellatus*, *Pleurotus salmoneo-stramineus*, *Pleurotus cornucopiae, Pleurotus dryinus*, *Pleurotus cystidiosus, Pleurotus spodoleucus, Pleurotus populinus, Pleurotus euosmus, Pleurotus nebrodensis, Pleurotus geesterani, Pleurotus geesterani* oder *Pleurotus ostreatus var. Columbinus,* geeignet.

### 2. Beispielhafte heterologe Expression des O-Methyltransferase codierenden Gens in E. coli

Das *O*-Methyltransferase codierende Gen wird nach gängiger Methode in das Plasmid pColdl für *E. coli* kloniert. Für die heterologe Expression des Proteins wird das Plasmid transformiert, z. B. in *E. coli* BL21 Gold (DE3) Zellen. Die Kultivierung erfolgt in geeignetem Medium z. B. in *lysogeny broth* (LB) Medium unter Verwendung von Carbenicillin (100 µg mL⁻¹) als Selektionsmarker bei 37 °C bis zu einer OD₆₀₀ von 0,4 - 0,6. Die Expression der *O*-Methyltransferase wird durch anschließende Zugabe von Isopropyl-*ß*-D-thiogalactopyranosid (finale Konzentration von 0,1 mM) induziert. Die Zellen werden für weitere 24 h bei 16 °C kultiviert, anschließend durch Zentrifugation geerntet (4000 g, 30 min, 4 °C) und bei -20 °C bis zur weiteren Verwendung gelagert.

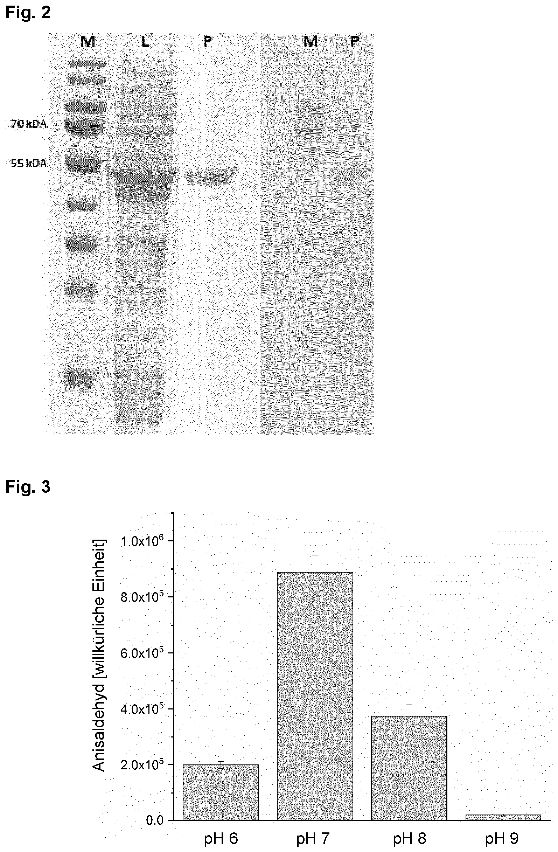

Für den Aufschluss werden die *E. coli* Zellen auf Eis aufgetaut, in Lysepuffer suspendiert (50 mM Phosphatpuffer, 300 mM NaCl, 5% Glycerol, pH 7,4) und mit Ultraschall aufgeschlossen (z. B. drei Zyklen für jeweils 60 s, 60 s Pause zwischen jeder Behandlung). Die Zellfragmente werden im Anschluss durch Zentrifugation (16.000 g, 30 min und 4 °C) separiert. Die Reinigung der O-Methyltransferase erfolgt mit üblichen Methoden, z. B. mittels FPLC unter Verwendung von einer Nickel *immobilized metal ion affinity chromatography* (IMAC). Die Entsalzung der mittels IMAC gewonnenen Proteinfraktionen erfolgt unter Verwendung von 100 mM PO₄³⁻-Puffer (pH 7,0) mittels *size exclusion chromatography* (SEC).
Die gereinigte und entsalzte *O*-Methyltransferase enthaltende Proteinfraktion wird mittels SDS-PAGE und Western Blot (Anti-His-Tag Antikörper) analysiert (vgl. Fig. 2). Die Proteinkonzentration wird mittels Bradford-Assay bestimmt (ca. 15 mg Protein je Liter *E. coli* Kultur).
Die vorstehend beispielhaft beschriebene Expression, Gewinnung und Reinigung der *O*-Methyltransferase kann durch die dem Fachmann allgemein bekannte Variationsmöglichkeit der Prozessparameter modifiziert werden, insbesondere im Rahmen einer Hochskalierung der Gewinnung des Enzyms und bei der Wahl des Organismus zur Expression.

### 3. Bestimmung des pH-Optimums

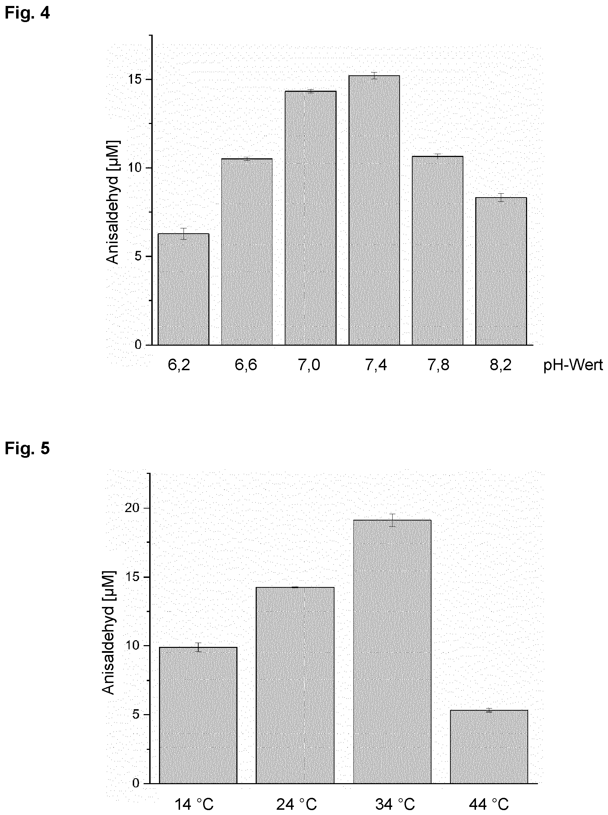

Bekanntermaßen katalysieren Biokatalysatoren, bzw. Enzyme wie *O*-Methyltransferasen, die von ihnen katalysierten Reaktionen bei bestimmten pH-Werten besonders effizient. Daher werden Versuche durchgeführt, um das pH-Optimum beispielhaft zu ermitteln. Die Versuchsergebnisse sind in Fig. 3 und Fig. 4 zusammengestellt. Das hier beispielhaft ermittelte Optimum liegt demzufolge in etwa bei pH 7,4. Auf Basis des für die Bildung von *p*-Anisaldehyd beschriebenen Bildungsweges wird beispielhaft *p*-Hydroxybenzaldehyd als Substrat (500 µM) gewählt. Der Cofaktor S-Adenosylmethionin (SAM) wird als Salz äquimolar mit dem Substrat eingesetzt. Die Umsetzungen werden in 20-mL-Headspacevials mit einem Reaktionsvolumen von 5 mL, bei 24 °C für 2 h in einem Überkopfschüttler (40 rpm) durchgeführt. Die Enzymkonzentration beträgt 0,16 µM.
Zunächst werden Umsetzungen in Davies-Puffer (Universalpuffer) der pH-Werte 2 - 12 durchgeführt. Nach erfolgter Umsetzung werden 200 µL 5 M Salzsäure und im Anschluss 2 mL Pentan/Diethylether-Gemisch (1:1,12; viv) zu den Ansätzen gegeben und für 10 min in einem Überkopfschüttler (40 rpm) extrahiert. Die organische Phase wird nach Separation und Trocknung über Natriumsulfat gaschromatographisch analysiert (vgl. Fig. 3).

Um den optimalen pH-Wert der Umsetzung noch näher zu spezifizieren, werden Umsetzungen in 0,1 M Phosphatpuffer (pH 6,2 - 8,2) durchgeführt. Nach erfolgter Umsetzung werden die Reaktionsansätze mit 100 µL 6 M Salzsäure versetzt und nach Membranfiltration für die HPLC-Messung an einer RP18-Säule eingesetzt (vgl. Fig. 4).

### 4. Bestimmung des Temperaturoptimums

Bekanntermaßen katalysieren Biokatalysatoren, bzw. Enzyme wie *O*-Methyltransferasen, die von ihnen katalysierten Reaktionen bei bestimmten Temperaturen besonders effizient. Daher werden Versuche durchgeführt, um das Temperaturoptimum beispielhaft zu ermitteln. Die Versuchsergebnisse sind in Fig. 5 zusammengestellt. Das hier beispielhaft ermittelte Optimum liegt demzufolge in etwa bei 34 °C.
Um die optimale Temperatur der Umsetzung näher zu spezifizieren, werden Umsetzungen in Phosphatpuffer (0,1 M, pH 7,4) zwischen 14 °C und 44 °C durchgeführt. Nach erfolgter Umsetzung werden die Reaktionsansätze mit 100 µL 6 M Salzsäure versetzt und nach Membranfiltration für die HPLC-Messung an einer RP18-Säule eingesetzt (vgl. Fig. 5).

### 5. Variation der Umsetzungsdauer

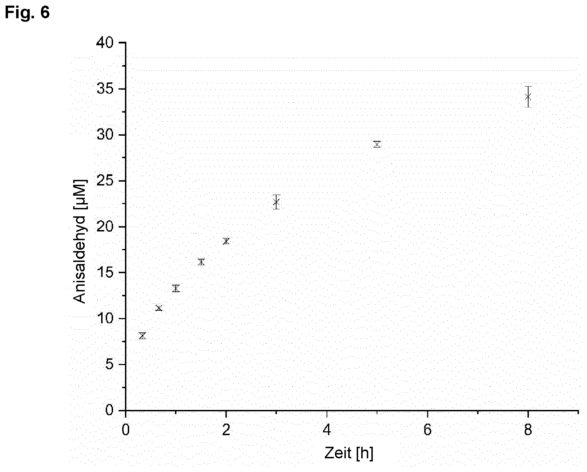

Bekanntermaßen gibt es für die Durchführung von mit Biokatalysatoren, bzw. Enzymen wie *O*-Methyltransferasen, katalysierten Reaktionen bei unterschiedlichen Temperaturen, pH-Werten und sonstigen Parametern jeweils optimale Umsetzungsdauern. Daher werden beispielhaft Versuche durchgeführt, um den zeitlichen Reaktionsfortschritt unter beispielhaft ausgewählten Reaktionsbedingungen zu ermitteln. Die Versuchsergebnisse sind in Fig. 6 zusammengestellt. Aus derartigen Ergebnissen lässt sich bekanntermaßen die Reaktionskinetik unter den jeweils gewählten Bedingungen berechnen.

Unter Anwendung der wie vorstehend beschrieben beispielhaft optimierten Parameter (0,1 M Phosphatpuffer (pH 7,4), 34 °C) werden Umsetzungen für 20 min, 40 min, 1 h, 1,5 h, 2 h, 3 h, 5 h und 8 h jeweils im Triplikat durchgeführt. Nach erfolgter Umsetzung werden die Reaktionsansätze mit 100 µL 6 M Salzsäure versetzt und nach Membranfiltration für die HPLC-Messung an einer RP18-Säule eingesetzt (vgl. Fig. 6). Über den gesamten beobachteten Zeitraum hinweg nimmt die Ausbeute mit zunehmender Umsetzungsdauer zu.

### 6. Umsetzung verschiedener hydroxylierter aromatischer Substrate

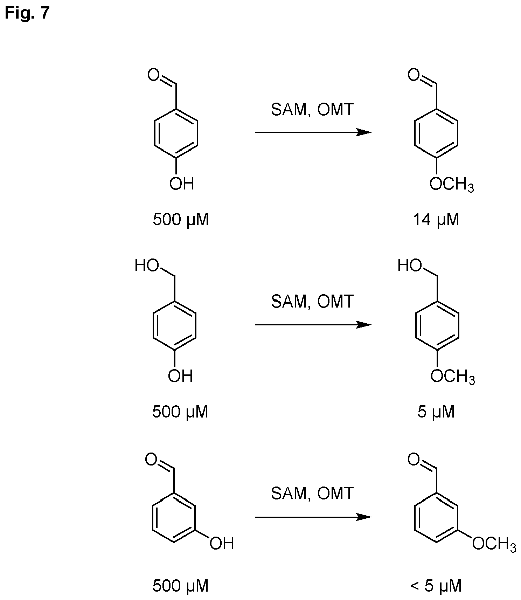

Bekanntermaßen weisen Biokatalysatoren, bzw. Enzyme wie *O*-Methyltransferasen, unterschiedliche Substratspezifitäten auf, d. h. sie können die Umsetzung verschiedener, mehr oder weniger unterschiedlicher Substrate katalysieren. Sie weisen somit ein Substratspektrum auf und katalysieren daher die Umsetzung verschiedener Substrate. Daher werden Versuche mit unterschiedlichen, beispielhaft ausgewählten, Substraten durchgeführt. Die Versuchsergebnisse (z. B. Fig. 7) zeigen, dass die *O*-Methyltransferase über ein breites Substratspektrum verfügt, d. h. die Umsetzung zahlreicher verschiedener Substrate katalysiert. Insbesondere ist das Substratspektrum der *O*-Methyltransferase Substanzklassenübergreifend, wie an der Umsetzung sowohl, von hydroxylgruppenhaltigen aromatischen Verbindungen als auch von thiolgruppenhaltigen Verbindungen erkennbar ist.

Unter Anwendung der bisher optimierten Parameter (0,1 M Phosphatpuffer (pH 7,4), 34 °C) werden beispielhaft Umsetzungen mit *m*- und *p*-Hydroxybenzaldehyd sowie *m*- und *p*-Hydroxybenzylalkohol durchgeführt (vgl. Fig. 7). Neben dem Triplikat der HPLC-Messung wird jeweils eine zusätzliche Probe mittels Pentan-Diethylether-Gemisch extrahiert und gaschromatographisch analysiert. Dem Fachmann ist bekannt, dass neben dem pH-Wert und der Temperatur weitere Optimierungsmöglichkeiten bestehen, beispielsweise Variation der Enzymkonzentration oder Verlängerung der Reaktionszeit.

### 7. Bestimmung enzymkinetischer Parameter

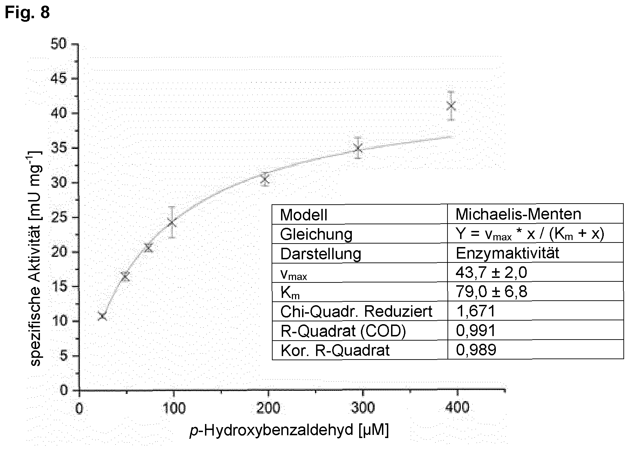

Bekanntermaßen werden Biokatalysatoren, bzw. Enzyme wie *O*-Methyltransferasen, auch durch die Bestimmung ihrer jeweiligen enzymkinetischen Parameter unter definierten Bedingungen charakterisiert. Daher werden anhand eines Beispiels auch die enzymkinetischen Parameter der *O*-Methyltransferase bestimmt. Die Versuchsergebnisse sind in Tabelle 1 zusammengestellt.
Mit dem Substrat, gegenüber dem die beispielhaft untersuchte *O*-Methyltransferase die höchste Aktivität aufweist, sowie dem Cofaktor werden die enzymkinetischen Parameter (Kₘ, vₘₐₓ) graphisch nach nichtlinearer Regression (Michaelis-Menten, Softwarepaket OriginPro 2020) ermittelt (vgl. Fig. 8, Fig. 8A, Fig. 8B). Zudem wird beispielhaft die katalytische Konstante (k_{cat}) sowie die katalytische Effizienz (k_{cat}/Kₘ) berechnet. Die resultierenden Werte sind zusammengefasst in Tabelle 1 aufgeführt.

**Tabelle 1: Enzymkinetische Parameter der aus P. sapidus abgeleiteten O-Methyltransferase unter Verwendung von p-Hydroxybenzaldehyd als Substrat**

| | |
|---|---|
| mittlere Michaelis-Menten-Konstante (Kₘ) | 79,0 µmol L⁻¹ |
| mittlere maximale Geschwindigkeit (vₘₐₓ) | 43,7 mU mg⁻¹ |
| katalytische Konstante (k_{cat}) | 2,5 s⁻¹ |
| katalytische Effizienz (k_{cat}/Kₘ) | 0,03 µmol⁻¹ s⁻¹ L |

### 8. Umsetzung des Thiol-Substrates 2-Methyl-3-furanthiol

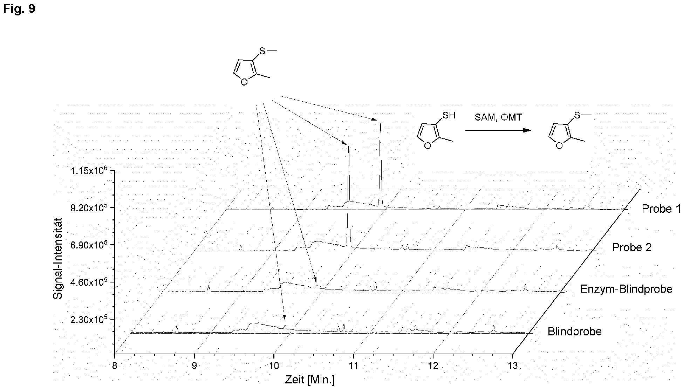

Durch die Umsetzung von 2-Methyl-3-furanthiol als Substrat mit *O*-Methyltransferase wird beispielhaft das breite, Substanzklassen-übergreifende, Substratspektrum der *O*-Methyltransferase gezeigt. Neben den unter 6. aufgeführten Substraten werden beispielhaft auch Umsetzungen unter Verwendung von 2-Methyl-3-furanthiol durchgeführt. Bei der Umsetzung wird im Vergleich zu den Blindwerten (chemisch sowie chemisch-biologisch, vgl. Legende zu Fig. 9) eine gesteigerte Bildung des methylierten Produktes mittels GC-MS nachgewiesen (vgl. Fig. 9).

### 9. Weitere Ausführungsbeispiele

In Tabelle 2 sind die Umsetzungsraten weiterer Ausführungsbeispiele zusammengestellt, wobei die Reaktionsbedingungen für die Umsetzungen in Tabelle 2 beispielhaft wie folgt ausgewählt wurden: Temperatur 34 °C, jeweils 500 µM Edukt- und Produktkonzentration, Phosphatpuffer pH = 7,4 (100 mM), Reaktionsdauer: 24 h, Durchführung jeweils ohne Schütteln).

**Tabelle 2: Weitere beispielhaft ausgewählte Umsatzraten**

| Edukt | Produkt | Produktkonzentration (Umsetzungsrate) |
|---|---|---|
| Vanillin | Veratrumaldehyd | 23,8 ± 0,9 µM (5%) |
| 6-Hydroxycoumarin | 6-Methoxycumarin | 11,5 ± 0,3 µM (2%) |

Dem Fachmann ist bekannt, dass die vorstehend beispielhaft genannten und variierten Parameter (etwa pH-Wert, Temperatur, Umsetzungsdauer) noch weiter variiert werden können, um die erzielbaren Ausbeuten noch weiter zu verbessern. Dem Fachmann ist ebenfalls bekannt, dass auch die Einbeziehung weiterer im Stand der Technik bekannter relevanter Parameter wie etwa die umgebende Gasatmosphäre, die Licht- und Druckverhältnisse ebenfalls noch variiert werden können, um das Verfahren zu optimieren, so dass entsprechende weitergehende Variationen vom Umfang der Erfindung sowie ihrer Äquivalente mit umfasst sind. Auch die ermittelten und offenbarten reaktionskinetischen Parameter (Michaelis-Menten-Konstante usw.) stellen keine Einschränkung des Verfahrens dar und sind beispielhaft zu verstehen.

Darüber hinaus ist fachkundigen Personen auch bekannt, dass die Enzyme hinsichtlich Proteinsequenz (Austausch, Einschub, Substitution von Aminosäuren) sowie auch dem Einbau nicht-natürlicher Aminosäuren modifiziert werden können, um sie umfangreichen weiteren potentiellen Edukten anzupassen, ohne dass dadurch der Umfang der Erfindung verlassen wird. Dies ermöglicht es, die erfindungsgemäße O-Methyltransferase im Rahmen des beanspruchten Schutzumfanges von wenigstens 70% Sequenzidentität mit SEQ-ID 1 oder SEQ-ID 2, bzw. bezüglich der Herkunft aus zahlreichen verschiedenen *Pleurotus spp.* für die Umsetzung vielfältiger weiterer Edukte anzupassen. Als weitere erfindungsgemäß verwendbare Edukte seien daher beispielhaft genannt: 3,4-Dihydroxybenzaldehyd, Isovanillin, Naringenin, Phloretin, Furaneol, Umbelliferon, Eugenol, Isoeugenol, Himbeerketon, *p*-Cumarsäure, Anthranilsäure.

Neben der Anpassung an verschiedenste Substrate, können in den beanspruchten Schutzumfang fallende Sequenzmodifikationen der *O*-Methyltransferase auch bewirken, dass die erfindungsgemäße *O*-Methyltransferase chemisch stabiler wird, wodurch die Einsatzbedingungen (Reaktionsbedingungen) ebenfalls noch deutlich variabler sein können, ohne den Umfang der Erfindung zu verlassen.
SEQ-ID 1: Aminosäuresequenz der *O*-Methyltransferase aus PSA-Transkriptom: 
MTTLSPSEGKAQIRALLNLINTSAEQAIAEYDKQECDIPSLTSGEPHPMDDRLPSLELKNT
LRILEGACAQLCVTLAPPAHTMLNYSMDVLVPSCISTVIQAGVAPLLAKHPKGLHIDVLSK
ETGIHPQKLATILRLLILNYCFQEVESNVFANNRLSLTLLPETSVVDILDLKTGEMHRKATL
WVYDALVDPDFGPTYDGNKSPLVYALRREGFDGSLYDYLQTQPGAVARFARAMLGFSV
SRGLMNLLNVFPWQELAPGSTVCDLGGGNGNTSIEIAKKFPHLKVHLQDLPDTIEEAKVF
WKEEYPDAIKDSRVAFTPIDFFKQAPVPDQDIYYISQIVHNWGDEDCITLLKNIRSAMSPK
SRLLINDYLASHLDKTSIANQHPSLPRAPYPLSPGFGRGMARTYTGDYTMLVVCNSRERS
LEDFIELCSAADLKFVRVWDLAETSVTEFVPA
SEQ-ID 2: Aminosäuresequenz der *O*-Methyltransferase in pColdl:

MTTLSPSEGKAQIRALLNLINTSAEQAIAEYDKQECDIPSLTSGEPHPMDDRLPSLELKNT
LRILEGACAQLCVTLAPPAHTMLNYSMDVLVPSCISTVIQAGVAPLLAKHPKGLHIDVLSK
ETGIHPQKLATILRLLILNYCFQEVESNVFANNRLSLTLLPETSVVDILDLKTGEMHRKATL
WVYDALVDPDFGPTYDGNKSPLVYALRREGFDGSLYDYLQTQPGAVARFARAMLGFSV
SRGLMNLLNVFPWQELAPGSTVCDLGGGNGNTSIEIAKKFPHLKVHLQDLPDTIEEAKVF
WKEEYPDAIKDSRVAFTPIDFFKQAPVPDQDIYYISQIVHNWGDEDCITLLKNIRSAMSPK
SRLLINDYLASHLDKTSIANQHPSLPRAPYPLSPGFGRGMARTYTGDYTMLVVCNSRERS
LEDFIELCSAADLKFVRVWDLAETSVTEFVPAHHHHHH

### Abbildungslegenden und Bezugszeichenliste

- Fig. 1:: Korrelation der Anzahl der Transkriptionskopien und der Peakflächen (normiert auf internen Standard) von p-Anisaldehyd der DK5 und MK57 Kulturen.
- Fig. 2:: SDS-PAGE-Gel der O-Methyltransferase (L: Lysat von *E. coli,* P: Protein nach Reinigung mittels FPLC) und Western Blot Membran (Anti-His-Tag Antikörper).
- Fig. 3:: Beispielhafte Umsetzung von p-Hydroxybenzaldehyd (500 µM) und SAM (500 µM) mittels *O*-Methyltransferase (0,16 µM) in Davies Puffer über 2 h (jeweils im Triplikat) zur Ermittlung des pH-Optimums; bei den für die Ordinatenachse gewählten willkürlichen Einheiten handelt es sich um die gerätetypische Quantifizierung der Peakfläche von *p*-Anisaldehyd im Chromatogramm der jeweiligen Probe aus der betreffenden Reaktionslösung nach Abschluss der Umsetzung.
- Fig. 4:: Beispielhafte Umsetzung von *p*-Hydroxybenzaldehyd (500 µM) und SAM (500 µM) mittels *O*-Methyltransferase (0,16 µM) in 0,1 M Phosphatpuffer über 2 h bei 24 °C (jeweils im Triplikat) zur weiteren Ermittlung des pHOptimums.
- Fig. 5:: Beispielhafte Umsetzung von *p*-Hydroxybenzaldehyd (500 µM) und SAM (500 µM) mittels *O*-Methyltransferase (0,16 µM) in 0,1 M Phosphatpuffer (pH 7,4) über 2 h (jeweils im Triplikat) zur Ermittlung des TemperaturOptimums.
- Fig. 6:: Beispielhafte Umsetzung von *p*-Hydroxybenzaldehyd (500 µM) und SAM (500 µM) mittels *O*-Methyltransferase (0,16 µM) in 0,1 M Phosphatpuffer (pH 7,4) bei 34 °C (jeweils im Triplikat) zur Ermittlung der optimalen Umsetzungsdauer.
- Fig. 7:: Beispielhafte Umsetzung verschiedener hydroxylierter Substrate (500 µM) und SAM (500 µM) mittels *O*-Methyltransferase (0,16 µM) in 0,1 M Phosphatpuffer (pH 7,4) bei 34 °C über 2 h (jeweils im Triplikat) als orientierende Untersuchung zur Eruierung des möglichen Substratspektrums.
- Fig. 8:: Beispielhafte Umsetzung von *p*-Hydroxybenzaldehyd und SAM (500 µM) mittels *O*-Methyltransferase (0,16 µM) in 0,1 M Phosphatpuffer (pH 7,4) bei 34 °C über 20 min (jeweils im Triplikat).

## Patentansprüche

1. Verfahren zum Herstellen mindestens eines Aromastoffes, wobei der wenigstens eine Aromastoff wenigstens eine -OCH₃-Gruppe und/oder wenigstens eine -SCH₃-Gruppe aufweist, mit mindestens einer *O-*Methyltransferase, wobei die mindestens eine O-Methyltransferase aus einer Subspezies der Gattung *Pleurotus* isoliert ist, und/oder eine Proteinsequenz aufweist, die zu wenigstens 70 % der Proteinsequenz von SEQ-ID 1 oder SEQ-ID 2 entspricht, umfassend die Schritte
a) Bereitstellung eines Umsetzungsmediums,
b) Einbringen von mindestens einem Edukt und der mindestens einen *O-*Methyltransferase in das Umsetzungsmedium aus Schritt a), wobei es sich bei dem mindestens einen Edukt um eine mono- oder polycyclische aromatische Verbindung mit mindestens einer Hydroxy- und/oder mindestens einer Thiolgruppe handelt,
c) Umsetzung des mindestens einen Eduktes unter Katalyse von der mindestens einen *O*-Methyltransferase zu dem mindestens einen Aromastoff.

2. Verfahren gemäß Anspruch 1, **dadurch gekennzeichnet, dass** es sich bei der *O*-Methyltransferase um ein aus einem Organismus isoliertes Enzym handelt.

3. Verfahren gemäß Anspruch 1 oder 2, **dadurch gekennzeichnet, dass** es sich bei der *O*-Methyltransferase um ein aus einem Basidiomyceten isoliertes Enzym handelt.

4. Verfahren gemäß Anspruch 3, **dadurch gekennzeichnet, dass** es sich bei der *O*-Methyltransferase um ein aus *Pleurotus sapidus* oder *Pleurotus eryngii* oder *Pleurotus pulmonarius* oder *Pleurotus sayor-caju* oder *Pleurotus floridanus* oder *Pleurotus albanus* oder *Pleurotus djamor* oder *Pleurotus ostreatus* oder *Pleurotus calyptratus* oder *Pleurotus citrinopileatus* oder *Pleurotus flabellatus* oder *Pleurotus salmoneo-stramineus* oder *Pleurotus cornucopiae* oder *Pleurotus dryinus* oder *Pleurotus cystidiosus* oder *Pleurotus spodoleucus* oder *Pleurotus populinus* oder *Pleurotus euosmus* oder *Pleurotus nebrodensis* oder *Pleurotus geesterani* oder *Pleurotus geesterani* oder *Pleurotus ostreatus var. Columbinus* isoliertes Enzym handelt.

5. Verfahren gemäß einem der vorangehenden Ansprüche 1 bis 4, **dadurch gekennzeichnet, dass** die *O*-Methyltransferase eine Proteinsequenz aufweist, die zu wenigstens 70 %, bevorzugt zu wenigstens 90 %, weiter bevorzugt zu wenigstens 95 % und ganz besonders bevorzugt zu wenigstens 99 %, der Aminosäuresequenz gemäß SEQ-ID 1 entspricht.

6. Verfahren gemäß Anspruch 2, **dadurch gekennzeichnet, dass** es sich bei der *O*-Methyltransferase um ein aus *E. coli* mittels Plasmid überexprimiertes Enzym handelt.

7. Verfahren gemäß Anspruch 6, **dadurch gekennzeichnet, dass** die *O-*Methyltransferase eine Proteinsequenz aufweist, die zu wenigstens 70 %, bevorzugt zu wenigstens 90 %, weiter bevorzugt zu wenigstens 95 % und ganz besonders bevorzugt zu wenigstens 99 %, der Aminosäuresequenz gemäß SEQ-ID 2 entspricht.

8. Verfahren gemäß einem der vorangehenden Ansprüche 1 bis 7, **dadurch gekennzeichnet, dass** das Edukt ausgewählt ist aus der Gruppe umfassend aromatische Verbindungen mit mindestens einer Hydroxy- und/oder mindestens einer Thiolgruppe.

9. Verfahren gemäß Anspruch 8, **dadurch gekennzeichnet, dass** das Edukt ausgewählt ist aus der Liste umfassend *m*-Hydroxybenzaldehyd, *p-*Hydroxybenzaldehyd, *m*-Hydroxybenzylalkohol, *p*-Hydroxybenzylalkohol, 2-Methyl-3-furanthiol, Vanillin, 6-Hydroxycumarin, 3,4-Dihydroxybenzaldehyd, Isovanillin, Naringenin, Phloretin, Furaneol, Umbelliferon, Eugenol, Isoeugenol, Himbeerketon, *p*-Cumarsäure, Anthranilsäure.

10. Verfahren gemäß einem der vorangehenden Ansprüche 1 bis 9, **dadurch gekennzeichnet, dass** der mindestens eine Aromastoff ausgewählt ist aus der Gruppe umfassend Methoxy- und Thioether-Verbindungen.

11. Verfahren gemäß Anspruch 10, **dadurch gekennzeichnet, dass** der mindestens eine Aromastoff ausgewählt ist aus der Liste umfassend *p-*Anisaldehyd, Vanillin.

12. Verfahren gemäß einem der vorangehenden Ansprüche, **dadurch gekennzeichnet, dass** Schritt c) des Verfahrens bei einem pH-Wert im Bereich von pH 6 bis pH 8 durchgeführt wird.

13. Verfahren gemäß einem der vorangehenden Ansprüche, **dadurch gekennzeichnet, dass** Schritt c) des Verfahrens bei einer Temperatur im Bereich von 10 °C bis 50 °C durchgeführt wird.

14. Verfahren gemäß einem der vorangehenden Ansprüche, **dadurch gekennzeichnet, dass** Schritt c) des Verfahrens über einen Zeitraum von 0,1 h bis 24 h hinweg durchgeführt wird.
